# EUROPÄISCHE PATENTSCHRIFT

(11) **EP 0 441 933 B1**
(45) Veröffentlichungstag und Bekanntmachung des Hinweises auf die Patenterteilung: **06.04.1994**
(21) Anmeldenummer: 90912905.8
(22) Anmeldetag: 24.08.1990
(51) Int. Cl.: C07D 239/26, C07D 239/34, C07D 213/57, C07D 213/65, C09K 19/34

(54) **DIFLUORBENZONITRILE UND FLÜSSIGKRISTALLINES MEDIUM**
DIFLUOROBENZONITRILES AND LIQUID-CRYSTAL MEDIUM
DIFLUOROBENZONITRILES ET MILIEU DE CRISTAUX LIQUIDES

(30) Priorität: 07.09.1989 DE 3929762; 30.01.1990 DE 4002609
(43) Veröffentlichungstag der Anmeldung: 21.08.1991
(73) Patentinhaber: MERCK PATENT GmbH, D-64271 Darmstadt (DE)
(72) Erfinder: REIFFENRATH, Volker, D-6101 Rossdorf (DE); KURMEIER, Hans-Adolf, D-6104 Seeheim-Jugenheim (DE); SCHEUBLE, Bernhard, D-6104 Seeheim-Jugenheim (DE)
(74) Vertreter: Schüttler, Reinhard, Dr.
(86) Internationale Anmeldenummer: EP9001412
(87) Internationale Veröffentlichungsnummer: WO9103468

(56) Entgegenhaltungen:
- EP-A- 0 097 033
- EP-A- 0 107 759
- EP-A- 0 195 974
- EP-A- 0 199 004
- EP-A- 0 240 320
- EP-A- 0 242 716
- EP-A- 0 317 175
- EP-A- 0 387 032
- WO-A-85/06373
- WO-A-86/06372
- WO-A-87/04158
- WO-A-87/04426
- WO-A-87/05018
- DE-A- 3 209 178

## Beschreibung

Die Erfindung betrifft neue. Difluorbenzonitrile der Formel I 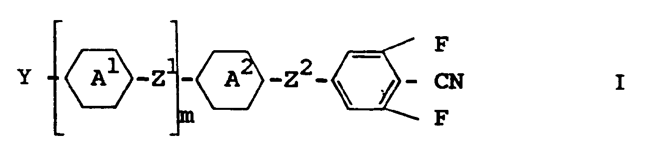
worin
- Y: Alkyl, Alkenyl, Alkoxy, Oxaalkyl oder Alkenyloxy mit jeweils 1 bis 12 C-Atomen,
einer der im Molekül vorhandenen Reste A¹ und A² 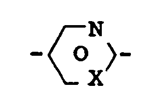 bedeutet, worin X CH oder N ist ,
und der andere Rest A¹ oder A² ausgewählt ist aus der Gruppe bestehend aus 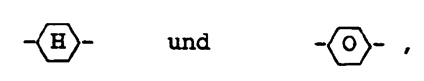 einer der Reste Z¹ und Z² eine Einfachbindung bedeutet, und der andere Rest Z¹ oder Z² - CH₂CH₂ - oder eine Einfachbindung ist,
und
- m: 0 oder 1 ist, mit der Maßgabe, daß im Falle m = 1, Z¹ = Z² = Einfachbindung und einer der Reste A¹ und A² = Pyrimidin-2,5-diyl und der andere Rest A¹ oder A² = 1,4-Phenylen, A¹ Pyrimidin-2,5-diyl ist.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine extrem hohe dielektrische Anisotropie bei gleichzeitig gutem Tieftemperaturverhalten.

Es wurde nun gefunden, daß Verbindungen der Formel I als Komponenten flüssigkristalliner Medien vorzüglich geeignet sind. Insbesondere verfügen sie über vergleichsweise niedere Viskositäten. Mit ihrer Hilfe lassen sich stabile flüssigkristalline Medien mit breitem Mesophasenbereich und vorteilhaften Werten für die optische und dielektrische Anisotropie erhalten.

Flüssigkristalle der Formel 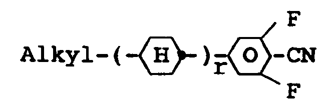
r = 1 oder 2
sind bereits aus DE 3209178 bekannt. Aus EP 0 317 175 sind Pyrimidinderivate der Formel 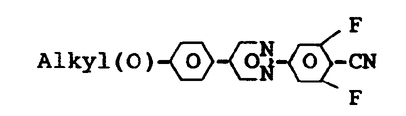
bekannt, die sich jedoch durch rechte hohe Schmelzpunkte auszeichnen.

Im Hinblick auf die verschiedensten Einsatzbereiche derartiger Verbindungen mit sehr hohem Δε war es jedoch wünschenswert, weitere Verbindungen zur Verfügung zu haben, die auf die jeweiligen Anwendungen genau maßgeschneiderte Eigenschaften, deutlich höhere Werte für Δε aufweisen als die aus DE 32 09 178 bekannten Verbindungen und günstigere Mesophasen, bessere Mischbarkeit mit anderen Flüssigkristallen und höhere thermische und UV-Stabilität als die aus EP 0 317 175 bekannten Verbindungen aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/ oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Die Verbindungen der Formel I umfassen dementsprechend Verbindungen der Teilformeln Ia bis Im:

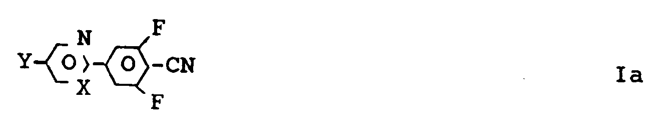

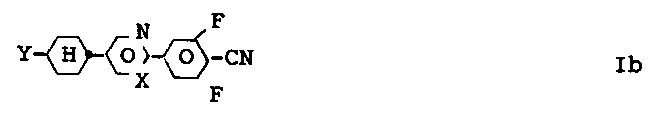

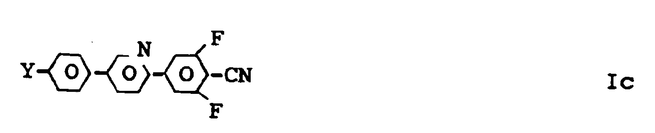

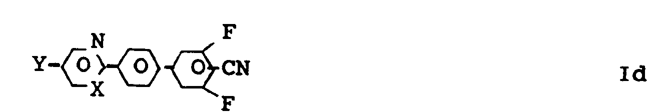

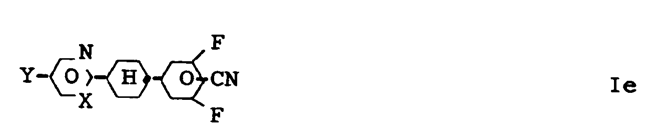

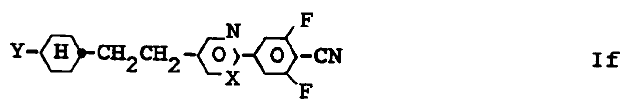

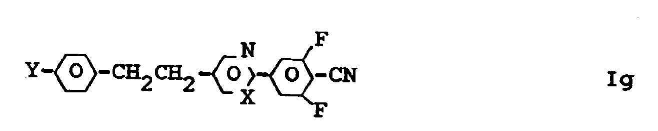

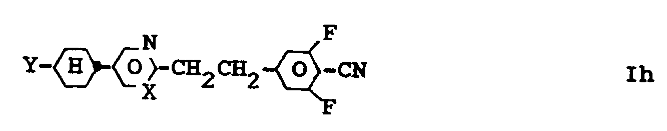

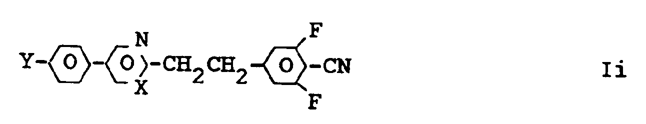

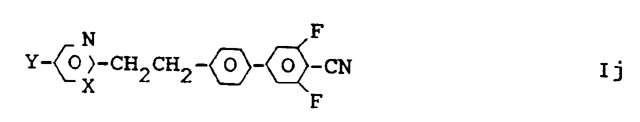

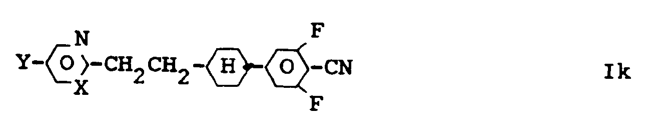

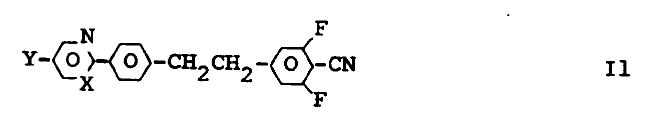

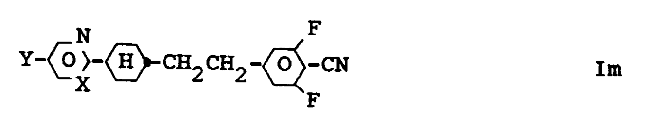

Darunter sind diejenigen der Formeln Ia und Id besonders bevorzugt.

Y bedeutet vorzugsweise Alkyl, ferner Alkoxy.

Vorzugsweise ist m = 1 oder im Falle m = 2 A¹ Pyrimidin-2,5-diyl und/oder einer der Reste Z¹ und Z² -CH₂CH₂-.

Falls Y einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4-oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls Y einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Verbindungen der Formeln I mit verzweigten Flügelgruppen Y können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste Y sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwahnten Varianten Gebrauch machen.

Die erfindungsgemäßen Verbindungen können z.B. hergestellt, indem man eine Verbindung der Formel II, 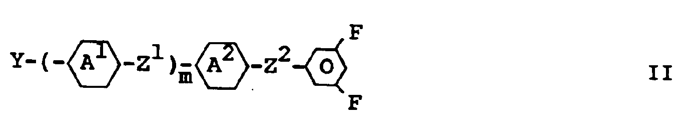
worin Y und Q die angegebene Bedeutung haben, gemäß folgendem Reaktionsschema metalliert und anschließend mit einem geeigneten Elektrophil umsetzt:

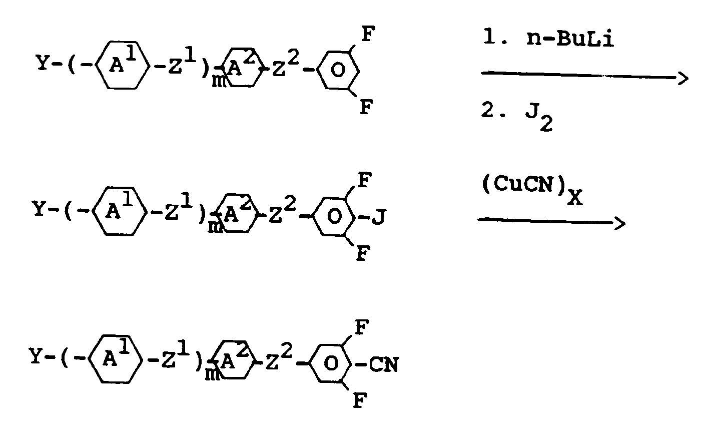

Weitere Synthesemethoden sind für den Fachmann augenscheinlich. Beispielsweise können in 5-Position entsprechend substituierte 1,3-Difluorbenzol-Verbindungen gemäß obigem Schema in die 2-Cyan-1,3-difluor-Verbindungen überführt werden und der Rest Y-Q- anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) angeführt werden.

Die Verbindungen der Formel II können beispielsweise nach folgenden Syntheseschemata hergestellt werden:

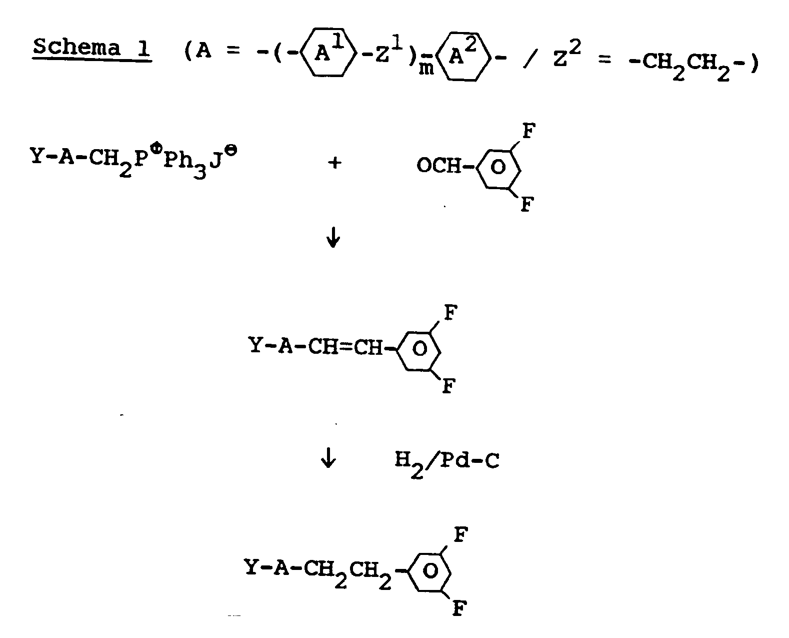

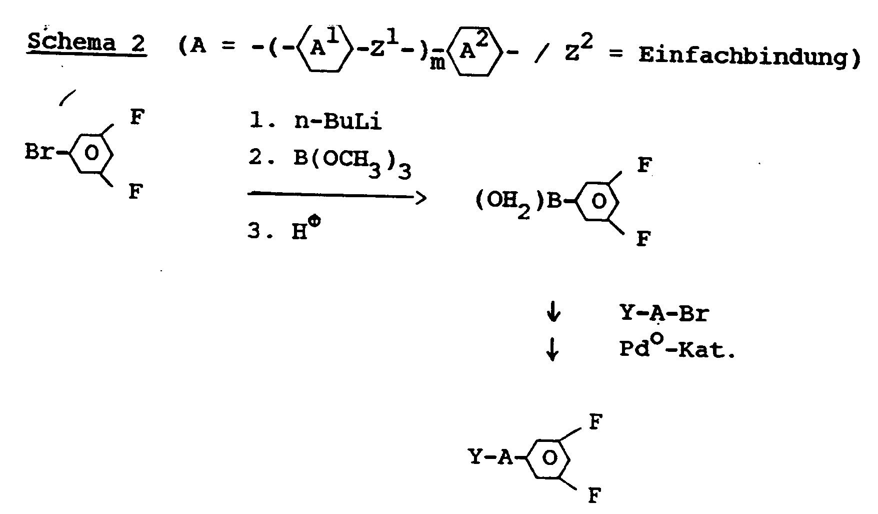

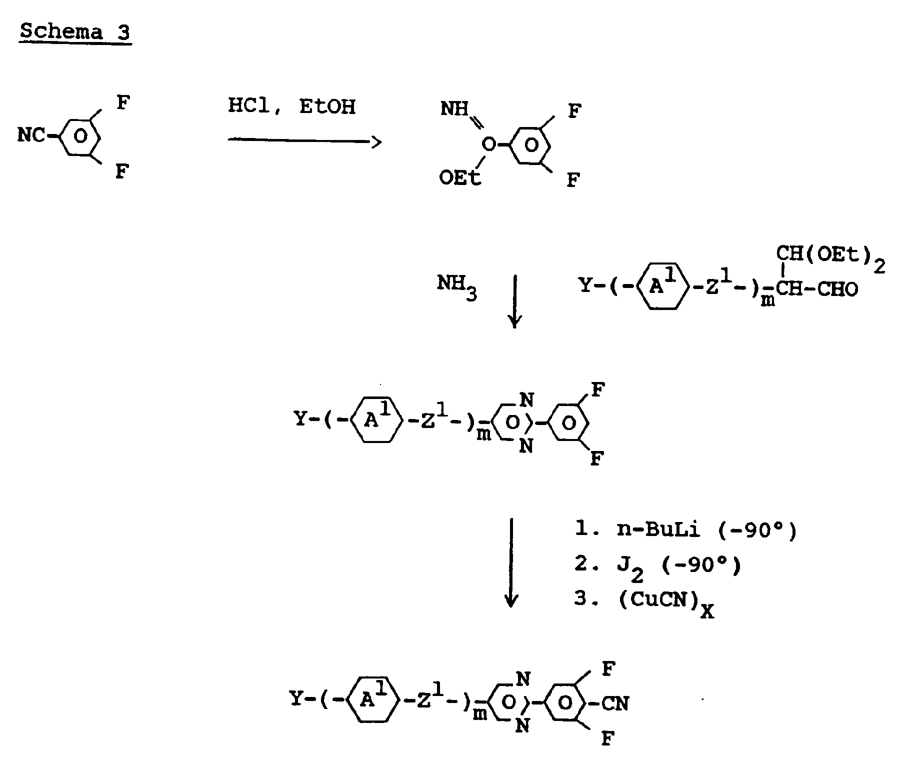

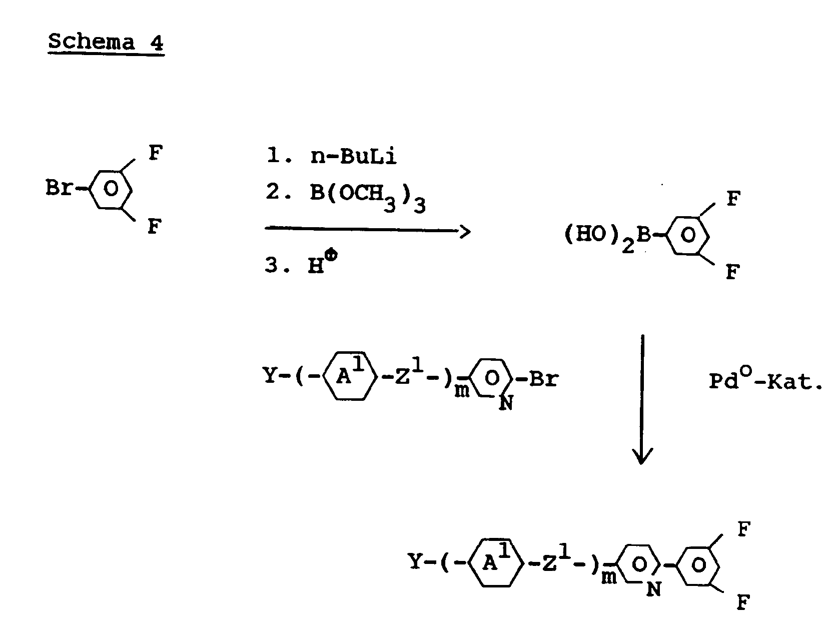

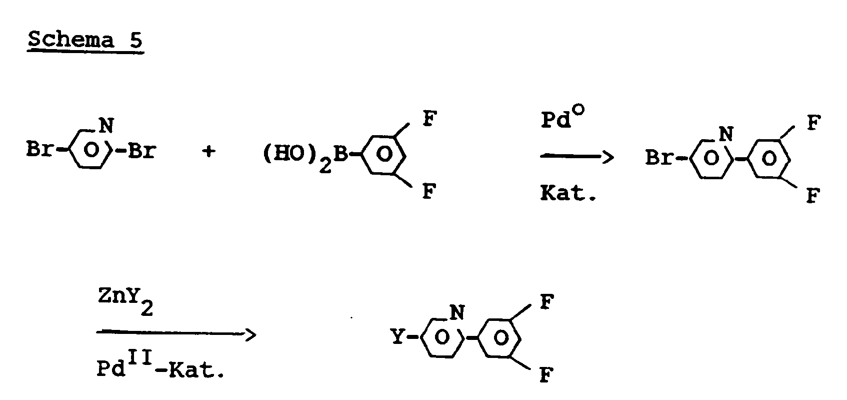

Die Ausgangsmaterialien sind entweder bekannt oder können in Analogie zu bekannten Verbindungen hergestellt werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesaure, Phenyl- oder Cyclohexylester der cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:
- R'-L-E-R'': 1
- R' -L-COO-E-R'': 2
- R' -L-OOC-E-R'': 3
- R'-L-CH₂CH₂-E-R'': 4
- R'-L-C≡C-E-R'': 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R'' voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R'' -F, -Cl, -NCS oder -(O)ᵢCH₃₋₍ₖ₊ₗ₎F_{K}Clₗ, wobei i 0 oder 1 und k+l 1, 2 oder 3 sind; die Verbindungen, in denen R'' diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R'' die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R'' -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %

wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Potenzangaben Gewichtsprozent. Alle Temperaturen sind in Grad Celsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- n-BuLi: n-Butyllithium
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure
- TMEDA: Tetramethylethylendiamin

### Beispiel 1

Eine Lösung von 0,1 m 1-[p-(5-n-Propylpyrimidin-2-yl)-phenyl)-2-(3,5-difluorphenyl)-ethan (hergestellt nach Schema 1) und 0,1 m TMEDA in 300 ml THF wird bei ca. -90° mit 0,1 m n-BuLi (1,5 M in Hexan) tropfenweise versetzt. Man rührt noch 30 min. bei dieser Temperatur und setzt dann eine Lösung von 0,1 m J₂ in 70 ml THF langsam zu. Nach beendeter Zugabe läßt man auf -20° erwärmen und hydrolysiert mit H₂O. Durch Zugabe von Diäthylether wird das Produkt vollständig in Lösung gebracht und überschüssiges J₂ durch Waschen mit Natriumthiosulfatlösung und H₂O entfernt. Nach dem Eindampfen verbleibt das Produkt als Rückstand und wird ohne weitere Aufreinigung mit 0,12 m (CuCN)ₓ und 100 ml NMP 4 h auf 170 °C im Ölbad erwärmt. Nach dieser Zeit wird das abgekühlte Reaktionsgemisch mit Wasser und CH₂CH₂ versetzt, die organische Phase gewaschen, getrocknet und eingedampft. Das reine Produkt läßt sich durch Chromatographie und Kristallisation gewinnen. Man erhält 1-[p-(5-n-Propylpyrimidin-2-yl)-phenyl]-2-(4-cyan-3,5-difluorphenyl)-ethan.

### Beispiel 2 bis 24:

Analog erhält man aus den entsprechenden Vorstufen der Formel II die folgenden erfindungsgemäßen Verbindungen:

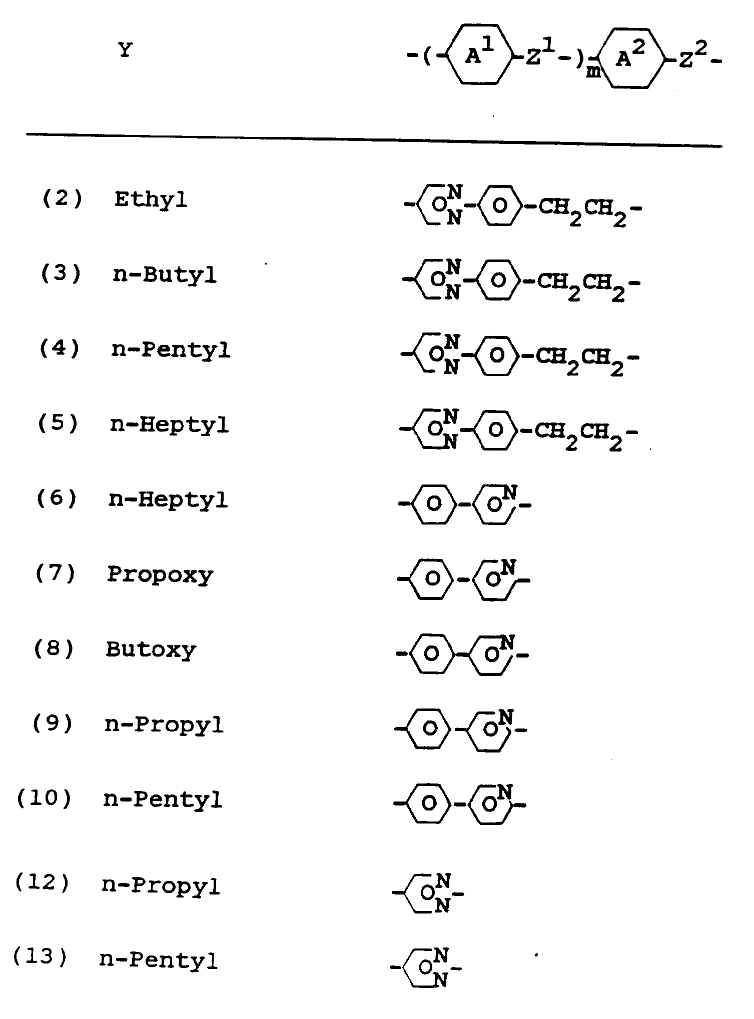

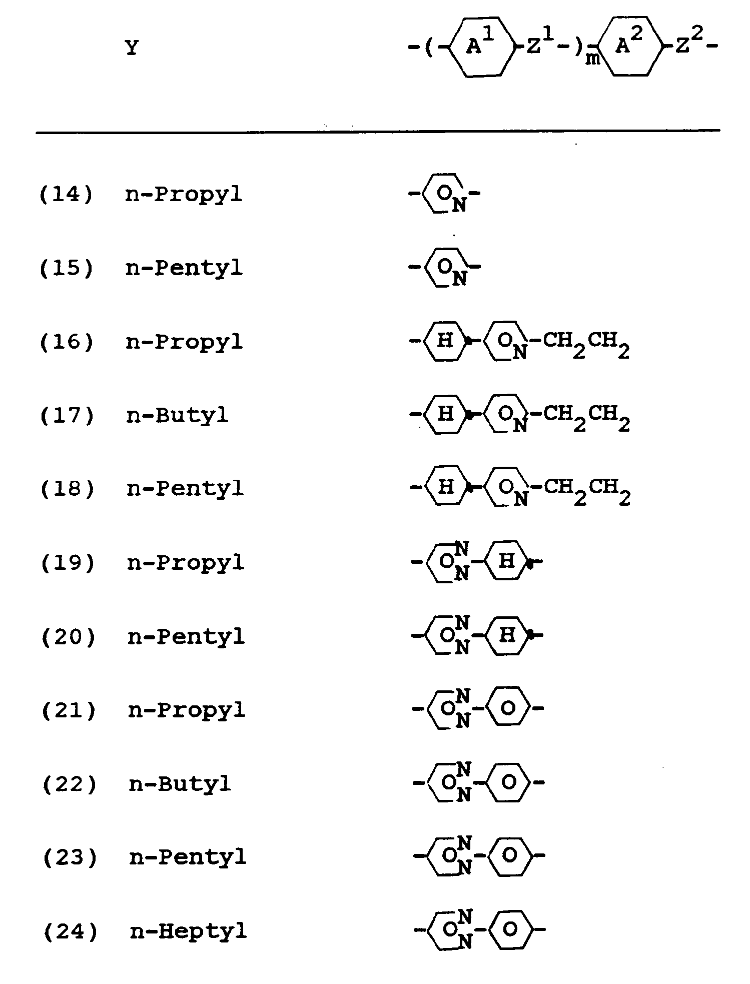

## Patentansprüche

1. Difluorbenzonitrile der Formel I 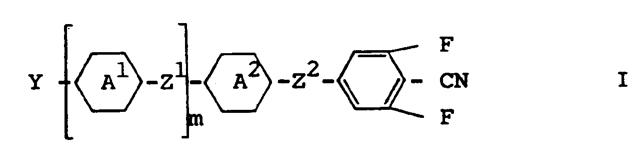 worin
Y Alkyl, Alkenyl, Alkoxy, Oxaalkyl oder Alkenyloxy mit jeweils 1 bis 12 C-Atomen,
einer der im Molekül vorhandenen Reste A¹ und A² 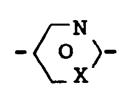 bedeutet, worin X CH oder N ist ,
und der andere Rest A¹ oder A² ausgewählt ist aus der Gruppe bestehend aus 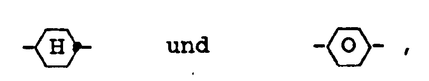 einer der Reste Z¹ und Z² eine Einfachbindung bedeutet, und der andere Rest Z¹ oder Z² - CH₂CH₂ - oder eine Einfachbindung ist,
und
m 0 oder 1 ist, mit der Maßgabe, daß im Falle m = 1, Z¹ - Z² = Einfachbindung und einer der Reste A¹ und A² = Pyrimidin-2,5-diyl und der andere Rest A¹ oder A² = 1,4-Phenylen, A¹ Pyrimidin-2,5-diyl ist.

2. Verwendung der Verbindungen der Formel I nach Anspruch 1 als Komponenten flüssigkristalliner Medien für elektrooptische Anzeigenelemente.

3. Flüssigkristallines Medium für elektrooptische Anzeigenelemente mit mindestens zwei flüssigkrisallinen Komponenten, dadurch gekennzeichnet, daß mindestens eine Komponente eine Verbindung der Formel I nach Anspruch 1 ist.

4. Elektrooptisches Anzeigenelement, dadurch gekennzeichnet, daß es ein Medium nach Anspruch 3 enthält.

## Claims

1. Difluorobenzonitriles of the formula I 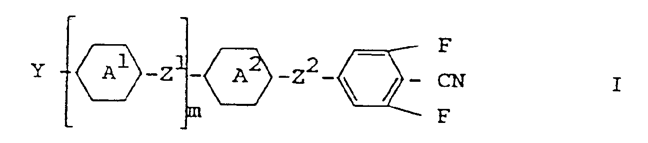 in which
Y is alkyl, alkenyl, alkoxy, oxaalkyl or alkenyloxy containing in each case 1 to 12 carbon atoms,
one of the radicals A¹ and A² present in the molecule is 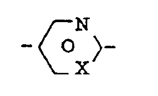 where X is CH or N,
and the other radical A¹ or A² is selected from the group comprising 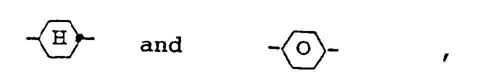 one of the radicals Z¹ and Z² is a single bond and the other radical Z¹ or Z² is - CH₂CH₂ - or a single bond, and
m is 0 or 1, with the proviso that if m = 1, Z¹ = Z² = single bond and one of the radicals A¹ and A² = 2,5-pyrimidinediyl and the other radical A¹ or A² = 1,4-phenylene, A¹ is 2,5-pyrimidinediyl.

2. Use of the compounds of the formula I according to Claim 1 as components of liquid-crystalline media for electro-optical display components.

3. Liquid-crystalline medium for electro-optical display components containing at least two liquid-crystalline components, characterized in that at least one of the components is a compound of the formula I according to Claim 1.

4. Electro-optical display component, characterized in that it contains a medium according to Claim 3.

## Revendications

1. Difluorobenzonitriles de formule I 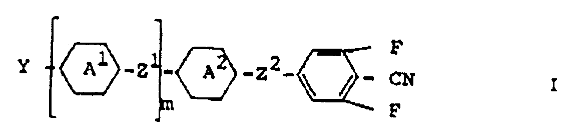 dans lesquels
Y représente un alcoyle, alcényle, alcoxy, oxaalcoyle ou alcényloxy avec à chaque fois de 1 à 12 atomes de carbone,
l'un des radicaux A¹ et A² présents dans la molécule représente 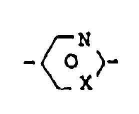 où X est CH ou N,
et l'autre radical A¹ ou A² est choisi dans le groupe constitué par 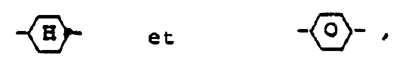 l'un des radicaux Z¹ et Z² représente une liaison simple, et l'autre radical Z¹ ou Z² représente - CH₂CH₂-ou est une liaison simple,
et
m vaut 0 ou 1, à la condition que dans le cas où m = 1, Z¹ = Z² = liaison simple et l'un des radicaux A¹ et A² = pyrimidine-2,5-diyle et l'autre radical A¹ ou A² = 1,4-phénylène, A¹ est un pyrimidine-2,5-diyle.

2. Application des composés de formule I selon la revendication 1 comme composants de milieux à cristaux liquides pour éléments d'affichage électro-optiques.

3. Milieu à cristaux liquides pour éléments d'affichage électro-optiques avec au moins deux composants à cristaux liquides, caractérisé en ce qu'au moins un composant est un composé de formule I selon la revendication 1.

4. Elément d'affichage électro-optique, caractérisé en ce qu'il contient un milieu selon la revendication 3.
